# EUROPÄISCHE PATENTSCHRIFT

(11) **EP 1 386 619 B1**
(45) Veröffentlichungstag und Bekanntmachung des Hinweises auf die Patenterteilung: **31.10.2007**
(21) Anmeldenummer: 03010245.3
(22) Anmeldetag: 07.05.2003
(51) Int. Cl.: A61L 2/07, A61L 2/24, A61M 5/00

(54) **Verfahren und Vorrichtung zur kontinuierlichen Messung, Erfassung und Regelung des Stützdrucks bei einer Dampfsterilisierung**
Method and device for the continuous measurement, aquisition and control of the compensation pressure during steam sterilisation
Méthode et dispositif pour la mesure, l'acquisition et le control en continu de la pression de compensation dans une stérilisation avec vapeur

(30) Priorität: 03.08.2002 DE 10235542
(43) Veröffentlichungstag der Anmeldung: 04.02.2004
(73) Patentinhaber: Arzneimittel GmbH Apotheker Vetter & Co. Ravensburg, 88212 Ravensburg (DE)
(72) Erfinder: Vetter, Udo. J, 88214 Ravensburg (DE); Treuer, Friedrich, 88212 Ravensburg (DE); Steigenberger, Klaus, 88239 Wangen (DE)
(74) Vertreter: Dziewior, Joachim

(56) Entgegenhaltungen:
- EP-A- 0 553 926
- DE-A- 1 933 542
- DE-A- 2 446 991
- US-A- 3 804 591
- VENTEN E ET AL: "EINE NEUE ANLAGE ZUR VERARBEITUNG VON SPRITZAMPULLEN" PHARMAZEUTISCHE INDUSTRIE, AULENDORF, DE, Bd. 40, Nr. 6, 1978, Seiten 665-671, XP008008584 ISSN: 0031-711X

## Beschreibung

Die Erfindung betrifft ein Verfahren zur kontinuierlichen Messung, Erfassung und Regelung des Stützdrucks bei der Sterilisierung von vorzugsweise in Behältnissen mit verschiebbarem Verschlußmechanismus angeordneten pharmazeutischen Produkten, insbesondere von mit Luft und/oder Flüssigkeit befüllten medizinischen Fertigspritzen, bei welchen der Verschlußmechanismus von einem Stopfen gebildet ist.

Weiter betrifft die Erfindung eine Vorrichtung zur Durchführung des Verfahrens.

Bei der Produktion bzw. Abfüllung von keimfrei in den Verkehr zu bringenden Produkten, insbesondere also bei pharmazeutischen Produkten, werden diese vor der Konfektionierung einer Sterilisierung unterworfen. Vorzugsweise handelt es sich dabei um Spritzen, die im vorabgefüllten Zustand einem Autoklavierungsprozeß, also einem Dampfsterilisationsverfahren unterworfen werden, um etwa noch vorhandene Keime abzutöten. Dieser Prozeß läuft abhängig vom jeweils abgefüllten Produkt etwa zwanzig bis sechzig Minuten lang bei einer Temperatur von etwa 120° Celsius und einem Kammerdruck von etwa 1,1 Bar ab. Die Spritzen, also das Sterilisationsgut, werden dabei zunächst bei Raumtemperatur in den Beschickungsraum des Autoklaven eingebracht. Anschließend erfolgt unter Zugabe von gesättigtem Wasserdampf eine kontinuierliche Erhöhung der Temperatur des Beschickungsraumes sowie des Sterilisationsgutes. Hieran schließt sich unter Beibehaltung einer Temperaturobergrenze für einen bestimmten Zeitraum die Sterilisationsphase an, worauf schließlich während einer Abkühlphase eine kontinuierliche Erniedrigung der Temperatur auf Raumtemperatur erfolgt.

Bedingt durch Unterschiede im Dampfdruck sowie dem insbesondere während der Aufheiz- bzw. Abkühlphase bestehenden thermischen Ungleichgewicht beobachtet man ein - unerwünschtes - Wandern des Stopfens im Spritzenzylinder.

Besteht das Sterilisationsgut aus geschlossenen Behältnissen wie Infusionsflaschen oder vorgefüllten Spritzen, so ist auf das sich im Inneren der Behältnisse während der Sterilisation einstellende Milieu zu achten. Entsprechend ist nach der Sterilisation dafür Sorge zu tragen, daß mit der fallenden Temperatur des Autoklaven der Überdruck im Inneren der Behältnisse nicht zu groß oder zu klein wird. Daher ist nach Möglichkeit die dem Sterilisationsgut vorlaufende Abkühlung des Beschickungsraumes des Autoklaven durch Applikation eines entsprechenden Stützdruckes - analog des durch Abkühlung reduzierten Druckes im Behältnis - auszugleichen. Dies dient insbesondere dazu, die Wanderung der Stopfen in den vorgefüllten Spritzen zu unterbinden.

Der Stützdruck wird daher gegenwärtig über die Temperatur und des sich durch die Sattdampfkurve ergebenden Druckes geregelt. Dieser ist dabei unter anderem auch abhängig von der im Sterilisationsgut abgefüllten Lösung. Dies bedeutet, daß je nach Zusammensetzung der abgefüllten Lösung aufgrund der unterschiedlichen Ausdehnungskoeffizienten der Befüllung ein separates Stützdruckprogramm erstellt und anhand von Versuchschargen bei festgestellter Stopfenwanderung entsprechend korrigiert werden muß. Dennoch ist immer wieder zu beobachten, daß insbesondere bei vorgefüllten Spritzen trotz Anwendung eines Stützdruckprogrammes eine Stopfenwanderung in den Spritzen auftritt. Die Stopfen befinden sich also nach Beendigung des Autoklavierungsprozesses nicht mehr an ihrer ursprünglichen Position.

Als Grund hierfür ist anzusehen, daß der Stützdruck aus der Sattdampfkurve des gesättigten Wasserdampfes ermittelt und der zusätzliche Stützdruck lediglich auf empirischen Erfahrungswerten beruht. Hinzu kommt jedoch auch, daß die Menge des Lufteinschlusses im Behältnis und die Gängigkeit der Gummistopfen im Spritzenzylinder zusätzlichen Einfluß haben.

Diese Stopfenwanderung hat insbesondere den Nachteil, daß beim nachfolgenden Konfektionierungsschritt, nämlich dem Einsetzen der Kolbenstange, diese nicht mehr ordnungsgemäß maschinell eingesetzt werden kann, wobei es insbesondere auch zum Bruck der Kolbenstangen kommen kann.

Aus der US-A-3804591 ist ein Verfahren bzw. eine Vorrichtung zur kontinuierlichen Messung, Erfassung und Regelung des Stützdrucks bei der Sterilisierung von Behältnissen bekannt. Der äußere Stützdruck wird kontinuierlich an den Innendruck angepasst.

Hiervon ausgehend liegt der Erfindung die Aufgabe zugrunde, ein Verfahren sowie eine Vorrichtung anzugeben, durch die eine präzisere Regelung des Stützdruckes ermöglicht wird, so daß Stopfenwanderungen ganz oder zumindest weitestgehend ausgeschlossen werden können.

Ein diese Aufgabe lösendes Verfahren ist dadurch gekennzeichnet, daß während der Sterilisierung der Druck im Inneren des durch den Verschlußmechanismus abgeschlossenen Behältnisses über einen die Verschiebung des Verschlußmechanismus aufgrund der Druckdifferenz zwischen dem Inneren und dem Äußeren des Behältnisses erfassenden Sensor ermittelt wird, und daß sodann aus diesem Meßsignal eine Regelgröße gebildet wird, über die der von außen auf das Behältnis einwirkende Stützdruck in der Weise angepaßt wird, daß der Innendruck und der Stützdruck gleich oder zumindest nahezu gleich sind.

Der durch die Erfindung erreichte Fortschritt besteht im wesentlichen darin, daß für die Regelung des Stützdruckes unmittelbar die Verhältnisse in dem Behältnis herangezogen werden, wobei dies durch schon geringste Anzeichen einer Stopfenwanderung erfolgen kann.

Nach einer ersten Ausgestaltung der Erfindung kann vorgesehen sein, daß die Verschiebung des Verschlußmechanismus optisch mittels einer das Behältnis durchdringenden Lichtschranke registriert wird. Dabei ist es weiter von Vorteil, wenn die Verschiebung durch beidseits des Verschlußmechanismus in Verschiebungsrichtung gesehen, jeweils eine Lichtschranke ermittelt wird, so daß sowohl der Einfluß von Druckerhöhung als auch -minderung erfaßt werden kann.

In vergleichbarer Weise besteht jedoch auch die Möglichkeit, daß die Verschiebung des Verschlußmechanismus durch Messung seines Abstandes von einem Sensor mittels Ultraschall oder auf optischem Wege registriert wird. Dies kann entweder durch eine reine Distanzmessung oder aber auch - insbesondere bei optischer Abtastung - durch Reflektion bei schrägem Lichteinfall erfolgen.

Zur Durchführung des Verfahrens wird eine Vorrichtung vorgeschlagen, bei der zur Druckermittlung im Inneren des durch den Verschlußmechanismus abgeschlossenen Behältnisses ein die Verschiebung des Verschlußmechanismus aufgrund der Druckdifferenz zwischen dem Inneren und dem Äußeren des Behältnisses erfassender Sensor vorgesehen ist, und daß eine Regeleinheit aus dem Meßsignal eine Steuergröße bildet, über die der von außen auf das Behältnis einwirkende Stützdruck in der Weise eingestellt wird, daß der Innendruck und der Stützdruck gleich oder zumindest nahezu gleich sind.

Soweit hier wiederum minimalste Verschiebungen des Verschlußmechanismus zur Steuerung des Stützdruckes herangezogen werden, sieht die Erfindung bei einem innerhalb eines Spritzenzylinders angeordneten und von einem Spritzenkolben gebildeten Verschlußmechanismus ein Meßelement vor, das über ein aus dem Spritzenzylinder vorstehendes Distanzstück am Spritzenkolben angeschlossen ist. Dies ermöglicht ein von der Art und Form des Verschlußmechanismus unabhängiges und somit zuverlässigeres Meßverfahren.

Das Meßelement kann hierbei als aktiver Meßsensor in Form eines Ultraschallsensors, eines Druckaufnehmers oder dergleichen oder auch als passives Element in Form eines Reflektors ausgebildet sein.

Im folgenden wird die Erfindung hinsichtlich des Meßprinzips an in der Zeichnung beispielhaft dargestellten Fertigspritzen näher erläutert; es zeigen:
- Fig. 1: eine Fertigspritze mit einer schematisch angedeuteten Meßmethode zur Ermittlung der Kolbenverschiebung,
- Fig. 2: ein weiteres auf der Kolbenverschiebung beruhendes Meßverfahren,
- Fig. 3: eine der Figur 2 ähnliche Meßanordnung,

Die Darstellungen in der Zeichnung dienen der Erläuterung eines Verfahrens zur kontinuierlichen Messung, Erfassung und Regelung des Stützdruckes bei der Sterilisierung von Behältnissen 4, die mit pharmazeutischen Produkten gefüllt und mit einem verschiebbaren Verschlußmechanismus 3 verschlossen sind. In der Zeichnung sind mit Luft 1 und Flüssigkeit 2 befüllte medizinische Fertigspritzen wiedergegeben, bei welchen der Verschlußmechanismus 3 von einem Stopfen gebildet ist. In vergleichbarer Weise ist die Erfindung jedoch auch bei anderen Behältnissen 4 wie beispielsweise Infusionsflaschen, anwendbar.

Entscheidend ist, daß während des Sterilisationsvorganges, bei welchem sich infolge der Temperaturerhöhung und anschließenden -erniedrigung die Druckverhältnisse im Inneren des Behältnisses 4 ändern, ein von außen wirkender Stützdruck so eingestellt bzw. geregelt wird, daß eine Kompensation in dem Sinne erfolgt, daß keine resultierenden, zu einer Verschiebung des Verschlußmechanismus 3 führenden Kräfte auf diesen einwirken.

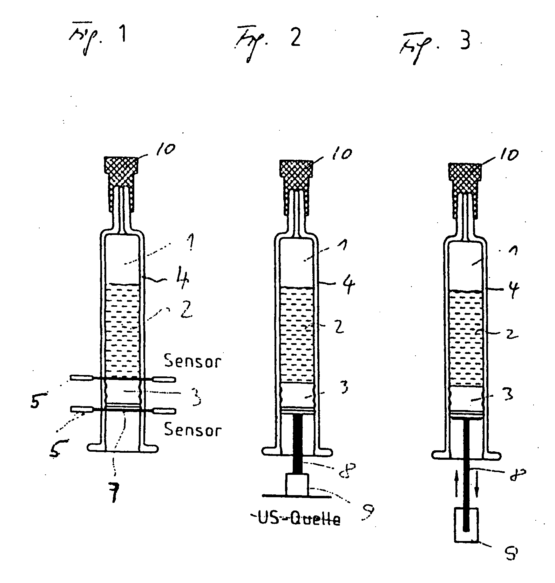

In den Figuren 1 bis 3 wird der während der Sterilisierung im Inneren des Behältnisses 4 auftretende Druck indirekt über eine Verschiebung des Verschlußmechanismus 3 aufgrund der Druckdifferenz zwischen dem Inneren und dem Äußeren des Behältnisses 4 erfassenden Sensor 5 ermittelt, wobei die Anordnung so empfindlich gewählt ist, daß schon geringste sich andeutende Verschiebungen erfaßt und ausgewertet werden.

Aus dem Meßergebnis wird dann in in der Zeichnung nicht näher dargestellter Weise eine Regelgröße gebildet, über die der von außen auf das Behältnis 4 einwirkende Stützdruck in einer Weise angepaßt wird, daß der Innendruck und der Stützdruck gleich oder zumindest nahezu gleich sind. Auch diese Regelung des Stützdruckes ist zeichnerisch nicht näher dargestellt; hierbei handelt es sich um aus der Praxis gängige Maßnahmen.

Erfolgt die Drucküberwachung gemäß den Figuren 1 bis 3, so kann zur Druckermittlung im Inneren des Behältnisses 4 ein solches aus der Produktionscharge herangezogen werden, da der Innenraum unbeeinflußt, also trotz der Meßanordnung steril bleibt.

Bei der Ausführungsform nach Figur 1 wird die Verschiebung des Verschlußmechanismus 3 optisch mittels einer das Behältnis durchdringenden Lichtschranke 7 registriert, wobei - wie sich unmittelbar erkennen läßt - beidseits des Verschlußmechanismus 3 jeweils eine Lichtschranke 7 vorgesehen ist, um die Verschiebung in beiderlei Verschiebungsrichtung erfassen zu können.

In den Figuren 2 und 3 ist an den Spritzenkolben 3 zunächst eine Distanzstück 8 angeschlossen, das aus dem Spritzenzylinder hervorsteht. Am Ende dieses Distanzstückes 8 ist ein Meßelement 9 angeordnet, bei dem es sich entweder um ein aktives Element in Form eines Ultraschallsensors, eines Druckaufnehmers oder dergleichen handeln kann oder aber auch um ein passives Element beispielsweise in Form eines Reflektors, das der Reflektion von gerade oder schräg auffallenden akustischen oder optischen Signalen dient. Hierfür sind dann in nicht näher dargestellter Weise optische oder akustische Empfänger vorzusehen, die aus der Laufzeit oder dem Ablenkwinkel die beginnenden Verschiebungen des Verschlußmechanismus 3 erfassen.

## Patentansprüche

1. Verfahren zur kontinuierlichen Messung, Erfassung und Regelung des Stützdrucks bei der Sterilisierung von in Behältnissen (4) mit verschiebbarem Verschlußmechanismus (3) angeordneten pharmazeutischen Produkten, insbesondere von mit Luft (1) und/oder Flüssigkeit (2) befüllten medizinischen Fertigspritzen, bei welchen der Verschlußmechanismus (3) von einem Stopfen gebildet ist und während der Sterilisierung der Druck im Inneren des durch den Verschlußmechanismus (3) abgeschlossenen Behältnisses (4) mittels eines Sensor (5) ermittelt wird und sodann aus diesem Meßsignal eine Regelgröße gebildet wird, über die der von außen auf das Behältnis (4) einwirkende Stützdruck in der Weise angepaßt wird, daß der Innendruck und der Stützdruck gleich oder zumindest nahezu gleich sind, **dadurch gekennzeichnet, daß** der Druck im Inneren des abgeschlossenen Behältnisses (4) über die Verschiebung des Verschlußmechanismus (3) aufgrund der Druckdifferenz zwischen dem Inneren und dem Äußeren des Behältnisses (4) ermittelt wird, wobei die Verschiebung optisch mittels einer das Behältnis durchdringenden Lichtschranke (7) oder durch Messung des Abstandes des Verschlußmechanismus (3) von einem Sensor (5) mittels Ultraschall oder auf optischem Wege registriert wird.

2. Verfahren nach Anspruch 1, **dadurch gekennzeichnet, daß** die Messung der Verschiebung des Verschlußmechanismus (3) über ein Reflektions- bzw. Dopplerverfahren erfolgt.

3. Verfahren nach Anspruch 1, **dadurch gekennzeichnet, daß** die Verschiebung durch beidseits des Verschlußmechanismus (3) in Verschiebungsrichtung gesehen jeweils eine Lichtschranke (7) ermittelt wird.

4. Vorrichtung zur kontinuierlichen Messung, Erfassung und Regelung des Stützdrucks bei der Sterilisierung von in Behältnissen (4) mit verschiebbarem Verschlußmechanismus (3) angeordneten pharmazeutischen Produkten, insbesondere von mit Luft und/oder Flüssigkeit befüllten medizinischen Fertigspritzen, bei welchen der Verschlußmechanismus (3) von einem Stopfen gebildet ist, wobei zur Druckermittlung im Inneren des durch den Verschlußmechanismus (3) abgeschlossenen Behältnisses (4) ein Sensor (5) vorgesehen ist und eine Regeleinheit aus diesem Meßsignal eine Steuergröße bildet, über die der von außen auf das Behältnis (4) einwirkende Stützdruck in der Weise eingestellt wird, daß der Innendruck und der Stützdruck gleich oder zumindest nahezu gleich sind, **dadurch gekennzeichnet, daß** die Verschiebung des Verschlußmechanismus (3) aufgrund der Druckdifferenz zwischen dem Inneren und dem Äußeren des Behältnisses (4) mittels einer das Behältnis durchdringenden Lichtschranke (7) oder durch Messung des Abstandes des Verschlußmechanismus (3) von einem Sensor mittels Ultraschall oder auf optischem Wege erfolgt.

5. Vorrichtung nach Anspruch 4, **dadurch gekennzeichnet, daß** bei einem innerhalb eines Spritzenzylinders angeordneten und von einem Spritzenkolben gebildeten Verschlußmechanismus (3) ein Meßelement (9) vorgesehen ist, das über ein aus dem Spritzenzylinder vorstehendes Distanzstück (8) am Spritzenkolben (3) angeschlossen ist.

6. Vorrichtung nach Anspruch 5, **dadurch gekennzeichnet, daß** das Meßelement (9) als aktiver Meßsensor in Form eines Ultraschallsensors, eines Druckaufnehmers oder als passives Element in Form eines Reflektors ausgebildet ist.

## Claims

1. Method for continuously measuring, recording and controlling the compensation pressure when sterilizing pharmaceutical products arranged in containers (4) with a movable closure mechanism (3), in particular of ready-to-use medical syringes filled with air (1) and/or liquid (2), in which the closure mechanism (3) is formed by a stopper and during the sterilization the pressure inside the container (4) sealed by the closure mechanism (3) is ascertained by means of a sensor (5) and there is then formed from this measured signal a controlled variable via which the compensation pressure acting from the outside on the container (4) is adjusted such that the internal pressure and the compensation pressure are the same or at least almost the same, **characterized in that** the pressure inside the sealed container (4) is ascertained via the displacement of the closure mechanism (3) on the basis of the pressure difference between the inside and the outside of the container (4), wherein the displacement is visually recorded by means of a light barrier (7) penetrating the container or by measuring the distance of the closure mechanism (3) from a sensor (5) by means of ultrasound or visually.

2. Method according to claim 1, **characterized in that** the measurement of the displacement of the closure mechanism (3) takes place via a reflection or Doppler method.

3. Method according to claim 1, **characterized in that** the displacement is ascertained by a respective light barrier (7) seen in each case in the direction of displacement on both sides of the closure mechanism (3).

4. Device for continuously measuring, recording and controlling the compensation pressure when sterilizing pharmaceutical products arranged in containers (4) with a movable closure mechanism (3), in particular of ready-to-use medical syringes filled with air and/or liquid, in which the closure mechanism (3) is formed by a stopper, wherein a sensor (5) is provided to ascertain the pressure inside the container (4) sealed by the closure mechanism (3) and from this measured signal a control unit forms an control variable via which the compensation pressure acting from the outside on the container (4) is adjusted such that the internal pressure and the compensation pressure are the same or at least almost the same, **characterized in that** the displacement of the closure mechanism (3) due to the pressure difference between the inside and the outside of the container (4) takes place by means of a light barrier (7) penetrating the container or by the measurement of the distance from a sensor by means of ultrasound or visually.

5. Device according to claim 4, **characterized in that** in the case of a closure mechanism (3) arranged inside the syringe cylinder and formed by a syringe piston, a measuring element (9) is provided for, which is attached to the syringe piston via a spacer (8) projecting out of the syringe cylinder.

6. Device according to claim 5, **characterized in that** the measuring element (9) is formed as an active measuring sensor in the form of an ultrasound sensor, a pressure recorder or as a passive element in the form of a reflector.

## Revendications

1. Procédé pour la mesure, l'acquisition et le contrôle en continu de la pression de compensation dans la stérilisation à la vapeur de produits pharmaceutiques contenus dans des récipients (4) équipés d'un mécanisme de fermeture (3) coulissant, en particulier de seringues médicinales remplies d'air (1) et/ou d'un liquide (2), ce mécanisme étant constitué par un bouchon, la pression à l'intérieur du récipient (4) obturé par le mécanisme de fermeture (3) étant déterminée, pendant la stérilisation, par un capteur (5) délivrant un signal de mesure à partir duquel est formée une grandeur de contrôle par laquelle la pression de compensation agissant de l'extérieur sur le récipient (4), est adaptée de manière que la pression interne et la pression de compensation soient égales ou au moins à peu près égales, **caractérisé en ce que** la pression à l'intérieur du récipient (4) obturé par le mécanisme de fermeture (3) est établie à partir du coulissement du mécanisme de fermeture (3) résultant de la différence des pressions existant à l'intérieur et à l'extérieur du récipient (4), ce coulissement étant enregistré optiquement au moyen d'une barrière lumineuse (7) traversant le récipient ou en mesurant la distance séparant le mécanisme de fermeture (3) d'un capteur (5), au moyen d'ultrasons ou sur le chemin optique.

2. Procédé selon la revendication 1, **caractérisé en ce que** la mesure du coulissement du mécanisme de fermeture (3) est effectuée par un procédé de réflexion ou à effet Doppler.

3. Procédé selon la revendication 1, **caractérisé en ce que** le coulissement est déterminé par deux barrières lumineuses (7) situées de part et d'autre du mécanisme de fermeture (3) selon la direction de coulissement.

4. Dispositif pour la mesure, l'acquisition et le contrôle en continu de la pression de compensation dans la stérilisation à la vapeur de produits pharmaceutiques contenus dans des récipients (4) équipés d'un mécanisme de fermeture (3) coulissant, en particulier de seringues médicinales remplies d'air (1) et/ou d'un liquide (2), ce mécanisme étant constitué par un bouchon, la pression à l'intérieur du récipient (4) obturé par le mécanisme de fermeture (3) étant déterminée, pendant la stérilisation, par un capteur (5) délivrant un signal de mesure à partir duquel est formée une grandeur de contrôle par laquelle la pression de compensation agissant de l'extérieur sur le récipient (4), est adaptée de manière que la pression interne et la pression de compensation soient égales ou au moins à peu près égales, **caractérisé en ce que** le coulissement du mécanisme de fermeture (3) résultant de la différence des pressions existant à l'intérieur et à l'extérieur du récipient (4), est enregistré optiquement au moyen d'une barrière lumineuse (7) traversant le récipient ou en mesurant la distance séparant le mécanisme de fermeture (3) d'un capteur (5), au moyen d'ultrasons ou sur le chemin optique.

5. Dispositif selon la revendication 4, **caractérisé en ce qu'**avec un mécanisme de fermeture (3) disposé à l'intérieur d'un cylindre de seringue et constitué par un piston de seringue (3), il est prévu un élément de mesure (9) qui est raccordé au piston (3) par une pièce d'espacement (8) sortant du cylindre de seringue.

6. Dispositif selon la revendication 5, **caractérisé en ce que** l'élément de mesure (9) est soit un capteur actif ayant la forme d'un capteur à ultrasons, d'un capteur de pression, soit un capteur passif ayant la forme d'un réflecteur.
